# EUROPEAN PATENT APPLICATION

(11) **EP 2 724 723 A1**
(43) Date of publication of application: **30.04.2014**
(21) Application number: 12189871.2
(22) Date of filing: 25.10.2012
(51) Int. Cl.: A61K 31/404, A61P 25/00, A61P 25/14, A61P 25/16, A61P 25/28

(54) **Tegaserod for use in the treatment of nerve injuries**

(71) Applicant: Universitätsklinikum Hamburg-Eppendorf, 20246 Hamburg (DE)
(72) Inventor: Schachner, Melitta, 20251 Hamburg (DE); Loers, Gabriele, 22529 Hamburg (DE)
(74) Representative: UEXKÜLL & STOLBERG

(57) **Abstract**

This invention relates to novel pharmaceutical composition comprising tegaserod and/or a pharmaceutically acceptable salt thereof for the treatment of neurological injuries, such as traumatic and chronic lesions of the central and peripheral nervous systems and for regenerating damaged nerves and nerve function. The pharmaceutical compositions of the invention can also be used for treatment and/or prevention of acute and chronic injuries to the central and peripheral nervous systems, for promoting growth and/or regrowth of nerves and enhancing formation of synapses and synaptic plasticity, and for neuroprotection.

SEQUENCE LISTING

<110>  Universitätsklinikum Hamburg-Eppendorf

<120>  TEGASEROD FOR USE IN THE TREATMENT OF NERVE INJURIES

<130>  P 88555

<160>  1

<170>  PatentIn version 3.3

<210>  1
<211>  12
<212>  PRT
<213>  Artificial

<220>
<223>  PSA mimetic peptide

<400>  1

Asn Thr His Thr Asp Pro Tyr Ile Tyr Pro Ile Asp
1               5                   10

## Description

This invention relates to novel pharmaceutical composition comprising tegaserod and/or a pharmaceutically acceptable salt or solvate thereof for the treatment of neurological injuries, such as traumatic and chronic lesions of the central and peripheral nervous systems and/or for regenerating damaged nerves and nerve function. The pharmaceutical compositions of the invention can also be used for treatment and/or prevention of acute and chronic injuries to the central and peripheral nervous systems, for promoting growth and/or regrowth of nerves and enhancing formation of synapses and synaptic plasticity, and for neuroprotection.

### BACKGROUND OF THE INVENTION

The treatment of complex spinal cord and other neurodegenerative injuries is a particularly challenging object of restorative medicine. The aim is to repair and functionally restore neural functions in the injured central and peripheral nervous systems. This includes the use of growth factors and adhesion molecules to promote axonal sprouting, activation of non-functional neurons and glia, thereby restoring a normal nervous system.

With respect to spinal cord injuries, recovery of locomotor function is one of the primary goals of research. Efforts to bridge spinal cord lesions using transplanted tissues are currently under investigation. However, there are so far no treatments available to reverse severe injury of the spinal cord which would lead to the regeneration of the central nervous system. The development of useful treatment approaches is hampered by the incomplete understanding of the molecular mechanisms which contribute to nervous tissue development, remodeling and repair. The ability of neurons and glial cells to modify cell surface interactions is a critical component of these processes. Among many candidate molecules that are potentially involved in such a process, isoforms of the neural cell adhesion molecule (NCAM), a member of the immunoglobulin superfamily, carrying an unconventional carbohydrate polymer, poly-α 2,8-sialic acid (PSA), are of particular interest. PSA is composed of negatively charged N-acetylneuraminic acid (sialic acid) residues in an alpha-2,8 linkage. A single PSA chain may comprise 50 or more monomers. However, the chain length may vary substantially in NCAM isolated from various sources (Rougon et al. (1993), Eur. J. Cell. Biol., 61: 197-207). NMR microscopy studies revealed that PSA has a helical structure in solution consisting of eight or more contiguous sialic acid units (Rougon et al., see above). In the hydrated state, PSA has a large volume and high negative charge density.

All the known alternatively spliced isoforms of NCAM can be polysialized at the fifth Ig-like domain (Rougon et al., see above). The attachment of PSA to NCAM is a developmentally regulated process; NCAM with a high PSA content is associated with morphogenetic changes during development such as cell migration, synaptogenesis and axonal growth and branching, while in adult brain poorly sialylated forms of NCAM are dominating. PSA-NCAM persists in adult brain structures that display a high degree of plasticity (Rougon et al., see above). For example, PSA-NCAM is required for two essential forms of activity-induced synaptic plasticity, long-term potentiation (LTP) and long-term depression (LTD) that are believed to be central to learning and memory as well as activity-dependent pattern formation during development (Muller et al. (1996), Neuron, 17:413-422).

Hippocampal tissue prepared from the NCAM mutant mice exhibited a markedly reduced capacity for LTP as well as LTD and this defect could be mimicked by the enzymatic destruction of the PSA moiety of NCAM. These observations indicate that PSA rather than the NCAM protein is required for plasticity. Morphological plasticity occurring in the hypothalamo-neurohypophyseal system is also dependent on the presence of PSA as an *in vivo* injection of endoneuraminidase negatively affects plasticity. PSA-NCAM is re-expressed in several pathological situations, such as muscle regeneration (Figarella-Branger et al. (1990), Cancer Res., 50: 6364-70) or brain neurodegenerative diseases (Le Gal La Salle et al. (1992), J. Neurosci., 12: 872-82). Based on these observations, PSA-NCAM emerged as an important permissive factor for dynamic changes in cell surface interactions required for morphogenesis and tissue remodeling.

PSA itself promotes cellular and axon/process motility in the nervous system by primarily decreasing homophilic interactions of NCAMs (Durbec and Cremer (2001), Mol. Neurobiol., 24(1-3): 53-64), but has also been implicated to mediate interaction of NCAMs with other molecules, such as HSPGs, BDNF, AMPA receptors, NMDA receptors, and histone H1 (Mishra et al. (2010), J. Neurosci., 30(37): 12400-13). The effects of PSA on promoting cellular migration and axon/process extension are thus of interest for the treatment of nervous system injuries and disorders. It has been shown that viral-induced expression of PSA enhances regeneration after spinal cord injury (Zhang et al. (2007), Mol. Ther., 15(10): 1796-804), promotes sensory neuron integration into injured spinal cord (Zhang et al. (2007), Molecular and Cellular Neuroscience, 35(1): 109-19), and increases Purkinje cell dendrite formation following injury (Zhang et al. (2007), Eur. J. Neuroscience, 25(2): 351-61). Overexpression of PSA on astrocytes improves axonal extension across spinal cord injuries (El Maarouf et al. (2006), PNAS, 103(45): 16989-94), and transplanted Schwann cells overexpressing PSA augment repair in both spinal cord and peripheral nerve. In light of these findings, PSA appears to represent one of the potential targets for therapeutic approaches directed to the promotion of plasticity and functional recovery after damage of the brain and/or nervous system.

Purification of PSA has turned out to be complicated (von der Ohe et al. (2002), Glycobiology, 12(1): 47-63) and the inherent short half-life of glycans in general have limited their usefulness in therapeutic interventions. In recent years, mimics and analogues of PSA have been identified and suggested for therapeutic use. For example, peptides have been isolated which mimic PSA epitopes, and it has been demonstrated that these PSA mimetic peptides are able to modulate cellular processes, which normally require polysialylation of NCAM, in a PSA-dependent manner (see, e.g., WO 2004/035609). The mimetic peptides showed a significant effect on axon growth, guidance and fasciculation, and it was also demonstrated that they influence neurons migration. The mimetic peptides were shown to be active *in vivo* at therapeutic doses without any toxicity.

For example, it was found that the PSA mimetic peptide having the sequence H-NTHTDPYIYPID-OH improved functional recovery following peripheral nerve injury when incorporated into a gel that was encapsulated in a conduit connecting the nerve stumps (Mehanna et al. (2009), Brain, 132 (6) : 1449-62). When applied through an osmotic pump, this peptide was also effective in promoting recovery after spinal cord injury (Mehanna et al. (2010), Mol. Ther., 18(1): 34-43). In these studies, the authors observed an increase in both the rate of recovery and myelination. A natural bacterial analogue of PSA, colominic acid, has also shown activity *in vitro* (Bruns et al. (2007), J. Biotechnol., 131(3): 335-45), but it likely faces the potential pitfall in that it is rapidly degraded by mammalian sialidases which are active in injury sites (Franz et al. (2005), J. Neurosci., 25(8): 2081-91).

Despite the progress that has recently been made in the development of PSA mimetic peptides, there is still a need for alternative compounds that mimic the function of PSA and improve nerve regeneration and/or motoneuron regeneration for potential use in the treatment of nerve injuries. It has now been surprisingly found that the 5-HT₄ agonist tegaserod represents a PSA mimetic compound that promotes neurite outgrowth and process extension of neurons and Schwann cells *in vitro.* Moreover, this compound also promoted nerve regeneration following injury to a peripheral nerve *in vivo.* Accordingly, tegaserod is a compound that is highly suitable for being used for treating nerve injuries.

Thus, the present invention provides pharmaceutical compositions comprising as an active agent the compound tegaserod or a pharmaceutically acceptable salt or solvate thereof. These compositions are for use in a method of treating or preventing nerve injury or for reducing the risk of nerve injury.

### DETAILLED DESCRIPTION OF THE INVENTION

Tegaserod is a compound that was approved by the FDA in 2002 for the treatment of irritable bowel syndrome and constipation (Muller-Lissner et al. (2001), Aliment. Pharmacol. Ther., 15(10): 1655-66). Tegaserod acts as agonist of the 5-HT₄ receptors on enteric neurons. Binding of tegaserod to the 5-HT₄ receptors promotes bowel movements (Liu et al. (2009), J. Neurosci., 29(31): 9683-99).

The present inventors have found that the tegaserod also mimics the functional activity of PSA. As described below in the Examples of the present invention, tegaserod was tested herein for its activity *in vitro,* and its PSA mimicking activity was confirmed on neurons and Schwann cells. In these cell types tegaserod promoted neurite and process extension to the same extent as a known PSA mimicking peptide and colominic acid. Maximal stimulatory effects of tegaserod were observed at 10-250 nM concentration, whereas maximal stimulatory effects were achieved with a concentration of 30 µM of the PSA mimicking peptide or 3 µM of colominic acid. These results show that tegaserod is about 20 times more effective than colominic acid and about 200 times more effective than the PSA mimicking peptide in stimulating process formation of neurons and Schwann cells. Overdosing of the PSA mimicking peptide or colominic acid did not lead to any negative side effects but at higher tegaserod concentrations the stimulatory effect was reduced or even abolished. Importantly, motoneurons lacking NCAM did not show enhanced neurite length from tegaserod or other PSA mimetics, which indicates that the mechanism of action of tegaserod is through NCAM. The neurite extending effects of tegaserod on cGNs were not observed upon use of serotonin or cisapride, which is a further evidence that the mode of action of tegaserod is independent of 5-HT (see Example 4 below).

*In vivo* studies were conducted in mice to test the potential of tegaserod to promote nerve regeneration following injury to a peripheral nerve. Tegaserod was delivered via a hydrogel within a conduit connecting the nerve stumps of the femoral nerve with a 2 mm gap. The results of this study showed that tegaserod was able to enhance repair of the femoral nerve. Tegaserod improved both the timeframe in which the foot base angle (FBA) began to recover as well as the FBA at the end of the 15-week recovery period. The timeframe for improvement in protraction limb ratio (PLR) was also improved after administration of tegaserod (see Example 5 below). Histology showed that tegaserod caused an increase in the number of axons in the regenerated nerve region as well as the percentage of axons that were myelinated. Mass of the quadriceps muscle innervated by the femoral nerve was also increased.

Tegaserod is an aminoguanidine derivative that is structurally similar to serotonin. Tegaserod binds with high affinity to the 5-HT₄ receptor and prevents serotonin from binding to it. The IUPAC name for tegaserod is (2E)-2-[(5-Methoxy-1H-indol-3-yl)methylene]-N-pentylhydrazine-carboximidamide. The chemical structure of tegaserod is the following:

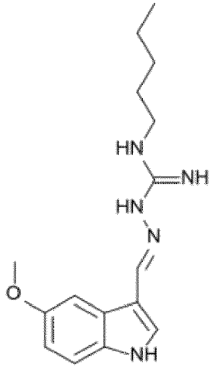

As the maleate salt, tegaserod is chemically designated as 3-(5-methoxy-1H-indol-3-ylmethylene)-N-pentylcarbazimidamide hydrogen maleate with the IUPAC name 1-[[(Z)-(5-methoxyindol-3-ylidene)methyl]amino]-2-pentylguanidine. The chemical structure is the following:

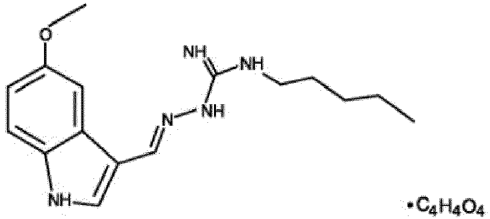

The chemical synthesis of tegaserod is described in detail in EP 505322 which is incorporated herein by reference. For the skilled person, it is evident that the above structure may be substituted in one or more than one positions or can be modified in another way as long as this substitution or modification does not affect the regenerative effect of the compound on damaged nerve tissue. For example, one or more hydrogen atoms of the C-H bonds of the heterocyclic ring system may be replaced without affecting the therapeutic and/or protective effect.

Apart from tegaserod, pharmaceutically acceptable salts of tegaserod can be used in the practise of the present invention. As used herein, a pharmaceutically acceptable salt of tegaserod refers to a salt which is suitable for administration into a subject, preferably a human subject, without undue toxicity. For example, the tegaserod salt should not induce any severe irritation or allergic responses. As used herein, pharmaceutically acceptable salts include acid addition salts that are formed with inorganic acids such as, for example, the hydrochloride, hydrobromide, hydrofluoride, hydrogen maleate, maleate, malonate, malate, sulphate or bisulphate, phosphate or hydrogen phosphate, acetate, citrate, fumarate, gluconate, lactate, succinate and tartrate salts. The use of the maleate or hydrogen maleate salt of tegaserod is especially preferred for medical use according to the invention.

Pharmaceutically acceptable solvates of tegaserod or its pharmaceutically acceptable salts, in particular of the hydrogen maleate salt, may also be used according to the invention, such as methanol, ethanol, isopropanol, dimethylsulfoxide (DMSO) or acetone solvates.

Tegaserod or its pharmaceutically acceptable salts or solvates are contemplated herein for use in a method of treating a disease or pathological condition which is characterized by the loss of functional neurons and/or Schwann cells. Preferably, the disease or condition is associated with the loss of neurons and/or Schwann cells in the peripheral nervous system. More preferably, the disease or condition is a lesion to the sensory neurons and/or motoneurons. Alternatively, the disease or condition is associated with the loss of neurons and/or Schwann cells in the central nervous system. Such acute or chronic disease or condition can, for example, be a lesion to the brain and/or spinal cord. In these types of conditions, tegaserod or its pharmaceutically acceptable salts or solvates thereof are useful for functional recovery by increasing axon survival or regeneration. According to a still further embodiment, the neurological disease is a lesion of the peripheral nervous system. According to a further preferred embodiment the neurological disease to be treated is age-related learning and memory problem. In this type of condition, tegaserod or its salts or solvates promotes nervous system plasticity by increasing neurogenesis and/or synaptic plasticity.

The lesions to the brain, spinal cord, sensory neurons and/or motoneurons can result from a surgical procedure, e.g. a surgery of the brain or spinal cord, or trauma, i.e. a physical injury, such as a traumatic damage of the spinal cord. A large proportion of neurological insults pertain to neurodegenerative insults.

In another embodiment, the disease to be treated by the administration of tegaserod or its salts or solvates is a neurodegenerative disease. As used in the context of the present invention, a neurodegenerative disease in which the number of specific cells of the brain or spinal cord is decreased, thereby leading to severe impairment of the nervous system. Here, the use of tegaserod or its pharmaceutically acceptable salt or solvate is expected to enhance axon outgrowth in the damaged areas of the brain or spinal cord. Preferably, the neurodegenerative disease to be treated according to the present invention is selected from the group consisting of Parkinson disease, Alzheimer's disease, Huntington's disease and Multiple Sclerosis.

In a still further embodiment the disease to be treated is a demyelinated disease. The demyelinated disease is preferably selected from the group of Charcot-Marie-Tooth disease (CMT), Guillain-Barré syndrome (GBS), schwannomatosis and chronic inflammatory demyelinating polyneuropathy (CIDP).

The disease or condition which is treated or prevented by tegaserod and/or the pharmaceutically acceptable salt thereof according to the invention is preferably selected from the group consisting of injury in the peripheral and/or central nervous system; injury inflicted by a surgical procedure, a disease and/or trauma; acute injury; chronic injury; injury inflicted by a degenerative disease; injury inflicted by a neurological disorder such as a neurodegenerative disease or a lesion, a brain or spinal cord injury, or a similar disease or condition. Since PSA has implicated in synaptic plasticity and many mental diseases have been attributed to be due to malfunctioning of synapses, it can be expected that tegaserod has several different therapeutic applications.

Tegaserod has been shown herein to be useful for inducing neurite outgrowth, for promoting process formation of glia cells, such as Schwann cells, for promoting proliferation of glia cells, such as Schwann cells, for enhancing migration and differentiation of neural progenitor cells (neural stem cells), for promoting survival of neurons, for promoting stem cell functions and/or promoting functional recovery of imperiled and/or injured neurons in acute and chronic injuries (including neurodegeneration) as well as enhancing synapse formation and synaptic plasticity. The pharmaceutical composition may also be for enhancing learning and memory, e.g., in treatment of diseases associated with deficiencies in learning and/or memory.

It has been shown in the present invention that the therapeutic effect of tegaserod which results in neurite outgrowth and axon survival does not depend on the binding to the 5-HT₄ receptor. Thus, the present invention contemplates the use of tegaserod or its salts or solvates for use in the treatment or prevention of diseases or conditions the pathology of which do not involve the 5-HT₄ receptor. In particular, the disease or condition to be treated according to the invention is not a gastrointestinal disease, such as irritable bowel syndrome.

In a further aspect, the present invention relates to a pharmaceutical composition comprising tegaserod and/or the pharmaceutically acceptable salt thereof. The pharmaceutical composition is for a use in the treatment of nerve injury or for the prevention of nerve injury.

The preparation of pharmaceutical compositions comprising tegaserod or pharmaceutically acceptable salts thereof as active ingredients is well known by those working in the field of pharmaceutics. Typically such compositions are prepared for injection either as liquid solutions or suspensions. Alternatively, the active ingredient may be emulsified in the compositions. The active ingredient can be mixed with excipients which are pharmaceutically acceptable and compatible with the active ingredient and in amounts suitable for use in the human patients.

In particular, the pharmaceutical compositions of the present invention preferably contain a physiologically acceptable carrier together with tegaserod or the salt thereof dissolved or dispersed therein as an active ingredient. As used herein, the term "pharmaceutically acceptable carrier" comprises, but is not limited to, water, saline, Ringer's Solutions, dextrose solution, and 5% human serum albumin. Liposome-based carriers and non-aqueous vehicles such as fixed oils may also be used. Further examples of suitable carriers for compositions comprising Tegaserod and/or pharmaceutically active salts thereof are described in standard textbooks, for example, in "Remington's Pharmaceutical Sciences", Mack Pub. Co., New Jersey (1991). In addition to the carrier, the composition may also contain further compounds, such as wetting agents, emulsifying agents, pH buffering agents, stabilizers, dyes and the like, insofar as these compounds do not interfere with the biological activity of tegaserod or the salts thereof.

The pharmaceutical composition provided by the present invention will be formulated to be compatible with the intended route of administration. Different routes of administration are feasible for providing tegaserod or the pharmaceutically active salts thereof to the site of neurological injury. Preferably, the pharmaceutical composition is formulated for oral administration, e.g. in the form of tablets, capsules, granule, powder, liquids, and the like. Alternatively, the pharmaceutical composition is formulated for parenteral administration, for example, for intravenous, intrathecal, intradermal, subcutaneous, topical, transmucosal, or rectal administration. The pharmaceutical composition may also be formulated for being administered by implantation, e.g. admixed with a three-dimensional carrier or scaffold, such as a hydrogel.

The pharmaceutical composition of the invention will preferably be formulated for oral administration, for example, in the form of granules, powders, tablets, pills, capsules, gelcaps, emulsions, suspensions or solutions. The preparation of solid dosage forms for oral intake can be achieved by mixing tegaserod or a salt thereof with an appropriate edible carrier, such as microcrystalline cellulose, methylcellulose, hydroxypropyl methylcellulose, casein, albumin, mannit, dextran, saccharose, lactose, sorbitol, agar, alginate, pectine, collagen, starch or gelatine. The solid dosage form may also include one or more excipients, such as surfactants, binders, thickeners, diluents, lubricants, disintegrants, and glidants. Further, the solid dosage form can comprise antioxidants (for example, ascorbic acid, tocopherol or cysteine), preservatives (for example, parabene), coloring or flavoring agents, and the like. Mixing of the above components may involve dry granulation, wet granulation, or direct compression.

Solutions or suspensions used for intravenous, intradermal or subcutaneous application usually comprise a sterile diluent such as water, saline solution, fixed oils, polyethylene glycols, glycerine, propylene glycol or other synthetic solvents. The solutions or suspensions may further comprise antibacterial agents such as benzyl alcohol or methyl parabens, antioxidants such as ascorbic acid or sodium bisulfite, chelating agents such as EDTA, buffers such as acetates, citrates or phosphates, and agents for the adjustment of tonicity such as sodium chloride or dextrose. The pH can be adjusted with suitable acids or bases, such as hydrochloric acid or sodium hydroxide. The parenteral preparation can be enclosed in ampoules, disposable syringes or multiple dose vials made of glass or plastic.

Pharmaceutical compositions suitable for injection normally include sterile aqueous solutions or dispersions and sterile powders for the extemporaneous preparation of sterile injectable solutions or dispersion. The composition intended for injection must be sterile and should be fluid in order to allow a convenient handling in a syringe. The composition should be stable under the conditions of manufacturing and storage and is preferably preserved against the contaminating action of microorganisms such as bacteria and fungi, for example, by including parabens, chlorobutanol, phenol, ascorbic acid, thimerosal, and the like into the composition. For intravenous administration, suitable carriers may comprise physiological saline, bacteriostatic water, Cremophor EL™ (BASF) or phosphate buffered saline (PBS). The carrier may also be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prolonged absorption of the injectable compositions can be achieved by including in the composition an agent which delays absorption, for example, aluminum monostearate or gelatin. Sterile injectable solutions can be prepared by incorporating the active ingredient (tegaserod and/or pharmaceutically acceptable salts thereof) in the required amount in an appropriate solvent with one or a combination of the above mentioned ingredients followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Administration of tegaserod and/or pharmaceutically acceptable salts thereof may also be achieved by transmucosal or transdermal delivery (Chen et al. (2006), Nature Biotechnology 24: 455-460. For transmucosal or transdermal administration, the pharmaceutical composition of the invention containing tegaserod or the salt thereof will comprise penetrants appropriate for the barrier to be permeated. Such penetrants are known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. Preferably, the compounds are be prepared in the form of suppositories, with conventional suppository bases such as cocoa butter and other glycerides or retention enemas for rectal delivery.

In one embodiment, the active compounds are prepared with carriers that will protect tegaserod and/or pharmaceutically acceptable salts thereof against elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparing controlled release formulation are well-known in the art. Furthermore, sustained-release compositions can be prepared. Suitable examples of sustained-release preparations include semi-permeable matrices of solid hydrophobic polymers, which matrices are in the form of shaped articles, e.g., films or microcapsules. Examples of sustained-release matrices include hydrogels, polyesters, polylactides, copolymers of L-glutamic acid and ethyl-L-glutamate, non-degradable ethylene-vinyl acetate, degradable lactic acid-glycolic acid copolymers and the like.

In a particularly preferred embodiment, tegaserod and/or pharmaceutically acceptable salts thereof is included into a hydrogel which is directly administered to the site of treatment, e.g., to the site of nerve injury. Hydrogels for the administration of active agents are known in the art and include, e.g., the PuraMatrix peptide hydrogel of Becton Dickinson. The hydrogel comprising tegaserod or the salt thereof can be applied during a surgical procedure which provides access to the site of treatment, e.g., during brain surgery.

Alternatively, the pharmaceutical composition of the invention may also be formulated for being administered by continuous infusion, e.g., using an Alzet osmotic pump, for example, for administering by continuous infusion for a period of between 2 and 21 days, more preferably a period of between 10 and 14 days. In a preferred embodiment, the pharmaceutical composition according to the invention is administered in a single bolus, preferably immediately after nerve injury has occurred or has been diagnosed. It may e.g., be administered within about 1-6 hours after occurrence or diagnosis of the nerve injury, more preferably within about 2-5 hours, most preferably within about 3 hours.

It is further preferred that the pharmaceutical composition is formulated for administration, e.g., by injection or administration, preferably locally, e.g., at the site of the lesion. If the injury is inflicted by an operation, or if there is a danger that an operation may inflict an injury to a nerve, the composition is preferably administered during the operation. It can e.g. be beneficial to administer the pharmaceutical composition of the invention in the context of prostate surgery/prostatectomy to reduce complications due to nerve injury, in the context of surgery near the spinal cord or brain surgery, e.g., due to a brain tumor. When injury due to a trauma is treated by an operation, this also opens the option of administration during the operation. Other options are infusion into the spinal cord or into the brain, e.g. by using an Alzet osmotic pump, or incorporation into a biomaterial with defined release properties that can control availability of the compound for up to several months. Alternatively, the pharmaceutical composition of the invention may be administered by injection, e.g., at the site of the lesion or by intravenous injection, or by infusion into the diseased tissue.

The pharmaceutical composition or the tegaserod and/or the pharmaceutically acceptable salt thereof according to the invention may also be administered before a lesion is formed or detected, to reduce the risk of nerve injury. Prevention of nerve injury can thus be a reduction of a risk of nerve injury. The invention in one aspect provides a method of reducing the risk of nerve injury, e.g., due to an operation, wherein the composition of the invention is administered at the site of the risk of nerve injury in the context of an operation, e.g., prostate surgery or brain surgery.

Tegaserod or the salt thereof or the pharmaceutical composition including same may be administered to the patient in need of treatment as a single administration. Alternatively, tegaserod or the salt thereof or the pharmaceutical composition may be administered according to different treatment courses comprising more than one administration to the patient, e.g., two, three, four or up to five administrations or more.

The pharmaceutical compositions of the invention will generally comprise tegaserod or the pharmaceutically acceptable salt thereof in a therapeutically effective amount. As used herein, a therapeutically effective amount means that tegaserod or the pharmaceutically acceptable salt or solvate thereof has to be present in an amount that is sufficient for treating the neurological disease or injury. Successful treatment may include alleviation of the signs, symptoms, or causes of a disease, or any other desired alteration of the patient's state of health. For example, in the event of a spinal cord injury, a therapeutically effective amount of tegaserod will at least partially restore the locomotor functions of the treated subject.

For example, the dosage of tegaserod or its salt or solvate per body weight can vary from about 0.0005 mg per kg body weight of the patient to about 100 mg per kg body weight of the patient, and preferably from about 0.005 mg per kg body weight of the patient to about 50 mg per kg body weight of the patient, used in one or more dose administrations daily. According to a particularly preferred embodiment of the invention, the therapeutically effective amount of tegaserod or its salt or solvate ranges from 0.01 mg per kg body weight of the patient to about 10 mg per kg body weight of the patient. The overall daily dosis of tegaserod or its salt or solvate is at least 1, 2, 3, 4, 5, 6, 7, 8, 9 or at least 10 mg per kg body weight of the patient.

It will be appreciated by those skilled in the art that the concrete amount of the tegaserod and/or pharmaceutically acceptable salt thereof to be administered to the patient will depend on several factors, such as age and weight of the patient, as well as on the nature and severity of the medical symptoms to be treated. The amount will in any individual case may be determined by one of ordinary skill in the art using routine experimentation.

The invention also provides a method for treating and/or preventing nerve injury, wherein a pharmaceutical composition comprising an effective amount of tegaserod and/or a pharmaceutically acceptable salt is administered to a patient in need thereof, preferably a patient suffering from a nerve injury.

### BRIEF DESCRIPTION OF THE FIGURE

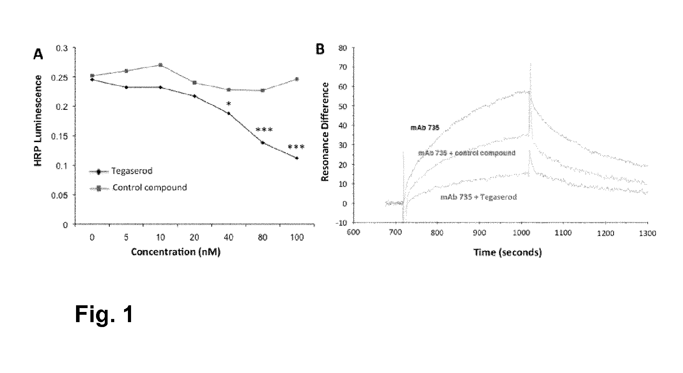

**Figure 1** shows that tegaserod competes with PSA mimicking peptides for binding to the anti-PSA antibody 735 (mAb735). (A) Results from a competition ELISA of the dose-dependent ability of tegaserod to interfere with binding of the anti-PSA 735 antibody with the PSA mimicking peptide adhered to the bottom of the wells, * p<0.05; *** p<0.005. (B) Surface plasmon resonance (SPR) profile of the effects of the presence of tegaserod to interfere with binding of mAb735 with the PSA peptide mimic covalently immobilized to a CM5 sensor chip. The SPR signal is displayed as resonance versus time, wherein 1000 RU (resonance units) represent a shift in resonance angle of 0.1° corresponding to a change in surface antibody concentration of approx. 1 ng/mm2.

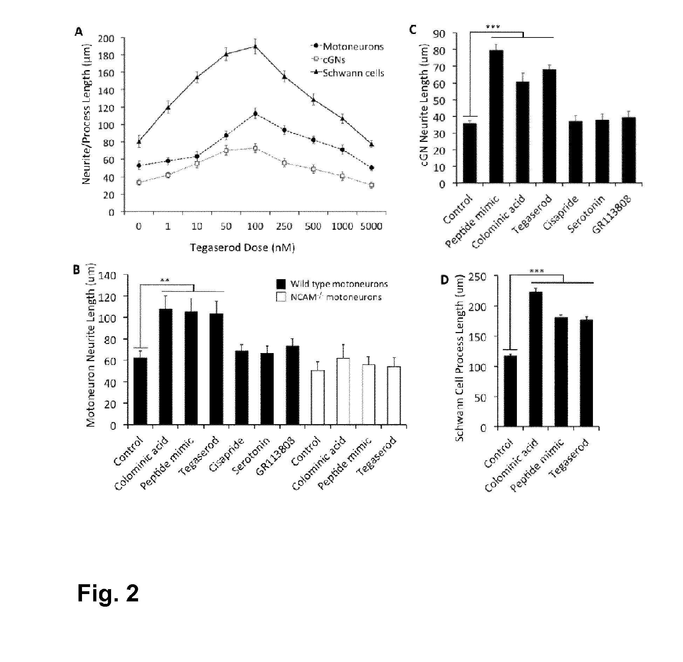

**Figure 2** shows the *in vitro* activity of tegaserod on extension of neurites/processes from motoneurons, cerebellar granule neurons (cGNs) and Schwann cells. (A) Dose response curve of tegaserod for the three cell types. (B) Comparison of the neurite-extending capabilities of tegaserod and related compounds on murine motoneurons. Tegaserod elicits neurite extension comparable to the known PSA mimetics of colominic acid and the peptide mimic, which is not observed in motoneurons isolated from NCAM-deficient mice. 5-HT agonists serotonin and cisapride as well as the 5-HT4 antagonist GR113808 did not cause an increase in neurite extension of motoneurons. (B-D) Concentrations of compounds, glycans and peptides: tegaserod, cisapride and GR113808 (100 nM), colominic acid (3 µM), PSA mimicking peptide (30 µM). All treatments were performed in duplicates and at least 100 cells were counted for each treatment. Results were at least confirmed in one or two more experiments. Mean values ± SEM are shown. (C-D) Effects of tegaserod and related compounds on cGNs and Schwann cells, *<0.05, **p<0.005, ***p<0.0005 (obtained by Student's t-test).

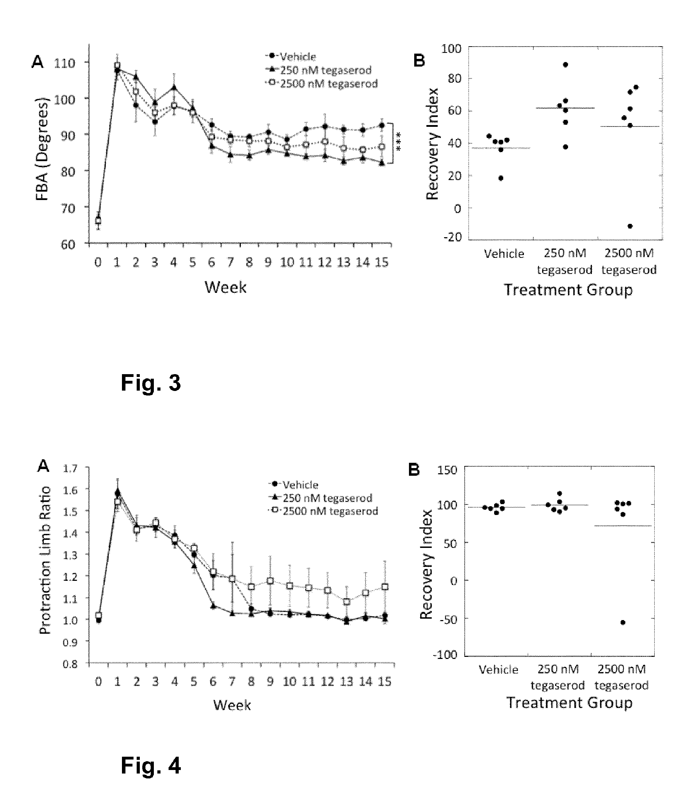

**Figure 3** shows the functional recovery of mice via the foot base angle (FBA) following bisection of the femoral nerve and insertion of a conduit containing vehicle alone, 250 nM tegaserod and 2,500 nM tegaserod. (A) FBA of mice that received vehicle, 250 nM tegaserod, or 2,500 nM tegaserod over a 15-week recovery period, ***p<0.005. (B) Recovery index (RI) of the FBA of individual animals within each treatment group.
**Figure 4** shows the functional recovery of mice via the protraction limb ratio (PLR) following femoral nerve injury. Following femoral nerve injury, extension of the injured hind limb is decreased relative to the healthy limb, with the PLR calculated by the length ratio of the healthy hind limb divided by that of the injured hind limb. A PLR of 1 indicates complete recovery of this hind limb function. (A) PLR of mice that received vehicle, 250 nM tegaserod, or 2,500 nM tegaserod measured over a 15-week recovery period. (B) RI of PLR of individual animals within each treatment group.

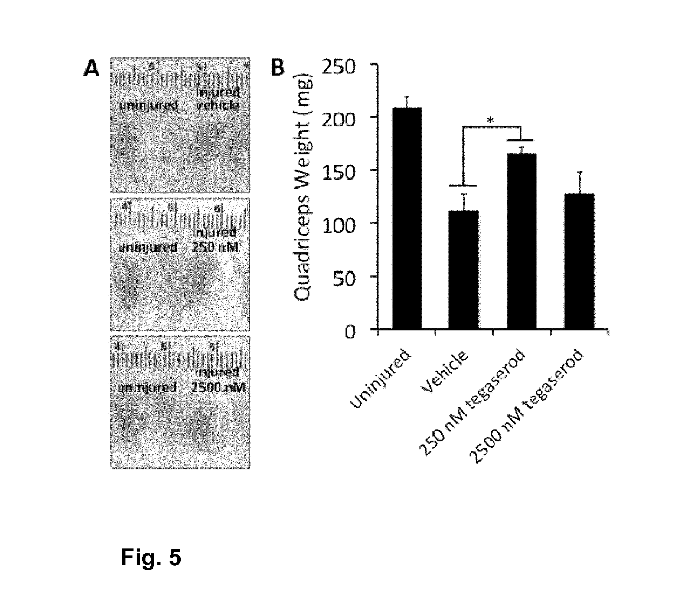

**Figure 5** shows the mass of quadriceps muscle collected from mice after 15-week recovery period. (A) Images of representative quadriceps muscles harvested from the healthy and injured hind limbs of mice following femoral nerve injury and insertion of a conduit containing vehicle or two doses of tegaserod. (B) Quantification of average muscle masses of mice that received vehicle, 250 nM tegaserod, or 2,500 nM tegaserod, * p<0.05.

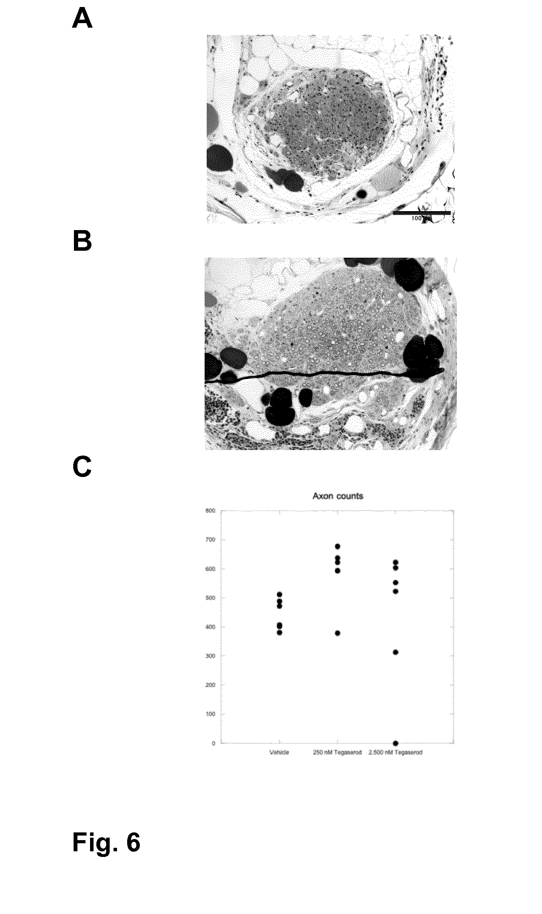

**Figure 6** shows the histological assessment of regenerated nerve treated with vehicle or tegaserod after a 15-week recovery period. (A) Representative image of a nerve cross section from a nerve treated with vehicle. (B) Representative image of a nerve cross section from a nerve treated with 250 nM tegaserod. Magnification 20x. (C) Quantification of the average number of axons contained within regenerated nerve that was fixed, stained with toluidine blue, and cut into 1 µm-thick sections, *p<0.05.

### EXAMPLES

The present invention will be further illustrated by the following examples. All animal experiments were conducted in accordance with the Rutgers Animal Care and Facilities Committee and the Institutional Animal Care and Use Committee (IACUC). It should be understood that these examples are provided by way of illustration only, and are not intended to limit the scope of the invention.

### EXAMPLE 1: Screening for organic PSA mimetic compounds

To identify organic PSA mimetics the NIH Clinical Collection 1 Library was screened. PSA mimicking peptide (NTHTDPYIYPIDC; Mehanna et al., (2009); Brain 132 (Pt 6): 1449-1462.) coupled to catalase (3 µg/ml; 25 µl/well) was immobilized on the surface of 384-well flat bottom microtiter plates with high binding surface (Corning Life Sciences, Amsterdam, The Netherlands). Wells were washed with PSA, blocked with 1% bovine serum albumin for 1 h at room temperature. 735 anti PSA antibody (0.1 µg/ml; 25 µl/well) was incubated with PBS, PBS containing DMSO solvent control or PBS containing 10 µM of the organic compounds from the library for 1 h at room temperature and then added to the wells coated with colominic acid coupled to catalase. As control for positive competition, 735 antibody was incubated with the PSA mimicking peptide (10 µM). After incubation for 1 h, wells were washed 10 times with PBS containing 0.05% Tween-20 (PBST), HRP-coupled secondary antibody (1:5,000 in PBS) was added for 1 h and after 10 more washes with PBST, binding of antibody was visualized by incubating wells with 25 µl of 0.4 mg/ml o-phenylenediamine dihydrochloride (Thermo Scientific) for 15 min. The reaction was stopped by addition of 25 µl 2.5 M H₂SO₄ and quantified using an ELISA reader (EnVision with Plateworks software, Perkin Elmer, Hamburg, Germany). Experiments were repeated three times to identify true hits.

Results: This randomized screening approach resulted in the identification of six organic compounds that could potentially mimic PSA. One of these compounds was tegaserod maleate.

### EXAMPLE 2: Competition ELISA using tegaserod

To confirm that the compounds identified in the screening from Example 1 are indeed PSA mimetics, binding of tegaserod maleate ((E)-but-2-enedioic acid; 1-[[(Z)-(5-methoxyindol-3-ylidene) methyl]amino]-2-pentylguanidine; hereafter termed tegaserod) and the control compound nitrendipine (5-O-ethyl 3-O-methyl-2,6-dimethyl-4-(3-nitrophenyl)-1,4-dihydropyridine-3,5-dicarboxylate) to the PSA antibody 735 was analyzed by competition ELISA. 384-well flat bottom microtiter plates (Corning Life Sciences) were coated in triplicates with 25 µl of PSA mimicking peptide coupled to catalase (3 µg/ml) or PBS alone at 4°C overnight. All following steps were carried out at room temperature. After blocking with 1% BSA in PBS for 1 h, the wells were washed three times for 5 min with PBS containing 0.05% Tween-20 (PBST). Wells were incubated with increasing concentrations of tegaserod and nitrendipine, pre-incubated with anti-PSA antibody 735 (0.1 µg/ml) for 1 h. After washing ten times with PBST, the wells were incubated with HRP-conjugated secondary anti-mouse antibody for 1 h, washed ten times with PBST, and binding was visualized by incubating wells with 50 µl of 0.4 mg/ml o-phenylenediamine dihydrochloride (Thermo Scientific) for 15 min. The reaction was stopped by addition of 50 µl 2.5 M H₂SO₄ and quantified using an ELISA reader (µQuant, Bio-TEK, Winooski, VT, USA) at 490 nm and the software KCjunior (Bio-TEK).

Results: Tegaserod was found to inhibit binding of mAb 735 with the PSA peptide mimic in a dose dependent manner (Figure 1A) with maximal inhibition occurring at approximately 100 nM. The control compound nitrendipine displayed no dose dependency to impede antibody binding relative to tegaserod.

### EXAMPLE 3: Surface Plasmon Resonance

The binding affinity of tegaserod to the PSA antibody was evaluated in a competition experiment by SPR measurements carried out on a BIAcore 3000 instrument (GE Healthcare Europe GmbH, Freiburg, Germany) with sensor chips maintained at 25°C. The running buffer was phosphate-buffered saline (PBS, pH 7.4). PSA mimicking peptide coupled to catalase was covalently immobilized to CM5 sensor chips (carboxymethyl dextran; GE Healthcare Europe GmbH) via primary amino groups, using standard coupling protocols (Karlsson et al. (1991); J. Immunol. Methods 145 (1-2): 229-240. In brief, the sensor surface was activated by a 7-min pulse of 0.2 M N-ethyl-N-(3-dimethylaminopropyl) carbodiimide and 50 mM N-hydroxysuccinimide. The PSA peptide solution (10 nM in 10 mM sodium acetate, pH 5.2) was then injected for 5-10 min. Ethanolamine (1 M, pH 8.5) was used to block remaining activated carboxyl groups. Ligand densities of 100-150 fmol/mm² were reached. Immobilized control peptide (10 nM PSA scrambled peptide coupled to catalase) was used as a reference surface. Regeneration of the sensor chip was achieved with injection of 10 mM glycine pH 2.5 at 10 ml/min. The SPR signal is displayed as resonance versus time where 1,000 RU (resonance units) represent a shift in resonance angle of 0.1° corresponding to a change in surface antibody concentration of ∼1 ng/mm². The data were analyzed using the BIAevaluation 3.0 software. All sensorgrams were corrected for background and bulk refractive index by subtraction of the reference.

To confirm that tegaserod binds specifically to antibody 735, a competition experiment was performed. First, the PSA specific monoclonal antibody 735 (10 nM) was pre-incubated for 1 h at room temperature with different molar concentrations (1, 2, 5, 8, 15 and 30 µM) of tegaserod or control compound. Then the antibody/organic compound solutions were injected (1 ml/l min) to the PSA peptide-coupled chip. The surface of the sensor chip was then regenerated with 10 mM glycine pH 2.5.

Results: Tegaserod disrupted the capability of mAb 735 to bind to the PSA peptide mimic bound to the chip surface by 80% compared to antibody that was not mixed with tegaserod (Figure 1B). Nitrendipine showed markedly less inhibition than tegaserod.

### EXAMPLE 4: In vitro analysis of neurite/process outgrowth

Primary cultures of cerebellar neurons or Schwann cells were prepared from cerebellum or dorsal root ganglia of 7-day-old C57BL/6J mice as described in Mehanna et al. (2009), Brain, 132 (Pt 6): 1449-62 and Loers et al. (2005), J. Neurochem., 92(6): 1463-76. Motoneurons were prepared from E14 C57BL/6J mice as described in Simova et al. (2006), Ann. Neurol., 60(4): 430-437. In brief, 48-well plates were coated with 0.01% poly-L-lysine (PLL) or poly-L-ornithine (PLO) overnight at 4°C, washed twice with water and air-dried. Directly before use, plates were washed twice with PBS, and Schwann cells, cerebellar neurons (PLL) or motoneurons (PLO) were seeded at a density of 1.25 x 10⁴ (Schwann cells and motoneurons) or 2.5 x 10⁴ cells (cerebellar neurons) per well in 250 µl of corresponding serum-free culture medium. Soluble organic compounds were added to the culture 1 h after seeding in increasing concentrations. After maintenance for 24 h at 37°C, cells were fixed with 2.5% glutaraldehyde and stained with 1% methylene blue/toluidine blue in 1% borax. Morphological quantification of neurite or process lengths was performed as described in Mehanna et al. (2009), see above. Schwann cell processes and neurites of cerebellar neurons with a length of at least one cell body diameter were counted and total neurite or process length per cell was determined by counting 50 cells in each of two wells per experiment. At least three independent experiments were performed for each culture. Also, the activity of tegaserod with the PSA peptide mimic and the bacterial PSA analogue, colominic acid, on neurite and process extension experiments was compared.

Results: A dose-response curve of tegaserod for PSA-responsive murine cerebellar granule neurons (cGNs), Schwann cells, and motoneurons revealed that tegaserod showed an effect on neurite and process extension of neurons and Schwann cells, respectively, at 1 nM and reached an optimal effect at 100 nM for all cell types (Figure 2A). At doses exceeding 100 nM to 250 nM, the effects of tegaserod on increasing neurite and process extension began to decline and reached control levels between 1 µM and 5 µM.

Colominic acid, the peptide mimic, and tegaserod all showed a significant stimulatory effect on neurite extension of murine motoneurons, while the 5-HT₄ agonist cisapride (Quigley et al. (2011), Journal of Digestive Diseases, 12(3): 147-156), serotonin, and the 5-HT₄ receptor antagonist GR 113808 (Gale et al. (1994), Br. J. Pharmacol., 111(1): 332-8) showed no ability to enhance motoneuron neurite extension (Figure 2B). Importantly, motoneurons isolated from NCAM^{-/-} mice showed no enhancement in neurite extension from colominic acid, the PSA peptide mimic, or tegaserod, suggesting that the effect of tegaserod on this population of motoneurons is NCAM dependent. The effects of tegaserod were also tested on a cerebellar granule neurons (cGNs) and Schwann cells and found to significantly enhance neurite/process outgrowth compared to control (Figure 2C, D).

### EXAMPLE 5: Effects of tegaserod on femoral nerve regeneration

Tegaserod was evaluated in a 2 mm femoral nerve defect of 12-week old C57BL/6J mice as previously described in Mehanna et al. (2009), see above. Briefly, mice were anesthetized and the femoral nerve of the left rear leg exposed. The nerve was bis-sected 3 mm proximal to the branching of the saphenous and motor branches, and the nerve ends were sutured 0.5 mm into a 3 mm polyethylene conduits with 0.58 mm inner diameter (Becton Dickinson), leaving an overall gap length of 2 mm. Conduits were filled with PuraMatrix peptide hydrogel (Becton Dickinson 354250) using 2x PBS as the gelation stimulant. Tegaserod was dissolved in the 2x PBS at concentrations of 500 nM and 5,000 nM, resulting in final concentrations of 250 nM and 2,500 nM once the PBS was combined 1:1 v/v with the PuraMatrix.

Locomotor recovery was quantified as described in Mehanna et al. (2009), see above, through assessment of the foot base angle (FBA) and protraction limb ratio (PLR). The FBA measures the angle between the hind paw and a beam as the mouse is pushing and bearing weight with the foot of the injured limb. In a healthy animal this angle averages approximately 65 degrees, whereas after injury, this angle is approximately 105 degrees. As the femoral nerve reinnervates the quadriceps muscle the FBA improves and will decline toward pre-injury levels (Irintchev et al. (2005), Eur. J. Neurosci., 22(4): 802-8).

The foot base angle (FBA) was measured prior to surgery and every week following surgery prior to sacrifice as previously described in Mehanna et al. (2009) and Simova et al. (2006), see above. FBA is governed largely by the quadriceps muscle, the major muscular innervation target of the femoral nerve. Mice were trained to walk on a beam for 2 weeks prior to surgery and single frame video motion analysis (Simi Reality Motion Systems, Simi Sportsplayer) was used to quantify the angle between the beam and the foot when the toes from the left leg are fully extended.

As a complement to assessment of the FBA that measures a weight-bearing reflexive movement, the protraction limb ratio (PLR) is indicative of voluntary movement of the hind limb. The PLR was similarly measured using single frame motion analysis (Irintchev et al. (2005), see above), and is taken as an indicator of voluntary movement. Mice are suspended by the tail above a pencil and allowed to grasp the pencil. The PLR is measured by dividing the relative length that the uninjured hind leg extends to the pencil by the length that the injured hind limb extends.

To account for any variability between animals, an index is calculated that takes into account the initial FBA and PLR of animals prior to surgery. The recovery index is calculated by the equation: *RI*= (*Xday y-Xday 1wk*)/(*Xday 0-Xday* 7) x 100, where *Xday0* is the measurement before injury, *Xday 1wk* is the measurement one week after injury, and *Xday y* is the measurement at the study endpoint. An RI value 100 indicates complete recovery of function.

Results: Analysis of the FBA showed that tegaserod improved the functional metric of regeneration. Prior to surgery and nerve bisection, the FBA of all mice was between 60 and 70 degrees (Figure 3C). A sustained improvement of the FBA in mice receiving 250 nM tegaserod-containing conduits first became apparent 6 weeks after the nerve injury and became statistically significant from the vehicle control group at 10 weeks. Animals that received the higher dose of tegaserod, 2,500 nM, trended toward improved FBA recovery but did not show statistically significant difference from vehicle control. At the end of the 15-week study, mice that had received 250 nM and 2,500 nM tegaserod exhibited an average FBA of 82 and 87 degrees respectively, compared to an average of 93 degrees for the control group.

Given some variation between individual animals, a recovery index (RI) for the FBA was calculated which normalizes the recovery at the end of the 15 weeks assessments to the initial FBA and the FBA measured at week 1, which is within 5-7 days of the surgery. The RI is expressed as a %, and the vehicle control group showed an average RI of 37%, with 5 of the 6 animals clustered between 36% and 42% (Figure 3D). Animals that received 250 nM tegaserod recovered to an average of 62%, while animals that had received 2,500 nM tegaserod only had an RI of 51%, with considerable variation evident for the animals having received 2,500 nM tegaserod.

Analysis of the PRL of mice with and without tegaserod suggested a positive influence at 250 nM tegaserod but not at 2,500 nM (Figure 4C). Vehicle control mice that received no tegaserod returned to pre-surgery PLR by 8 weeks, compared with 6 weeks for mice receiving 250 nM tegaserod. In contrast, mice that received 2,500 nM tegaserod exhibited PLR that remained consistently higher than the other two groups. Calculation of the RI for each mouse revealed that although the majority of mice with 2,500 nM eventually did recover to an RI of 100%, a single mouse within this group did not recover to pre-surgical PLR functional performance (Figure 4D).

### EXAMPLE 6: Determination of quadriceps muscle mass

The quadriceps muscle, which is solely innervated by the femoral nerve, was extracted from each mouse and weighed at the end of the 15 week recovery period. The quadriceps muscles were removed from the injured and uninjured hind limbs of the mice. The quadriceps femoris muscle was identified by first removing the skin overlying the cranial thigh. The muscle was bluntly dissected starting distally at the level of the patellar tendon, working proximally. Once loosened from the surrounding muscles and fascia, the muscle was cut free from its attachments from the patellar tendon distally and from the pelvis and femur proximally and fixed in buffered formalin. Quadriceps muscles are blotted dry using a paper towel and the mass was weighed using a Mettler Toledo XS105 Dual Range analytical balance.

Results: The muscle mass of quadriceps of the non-injured legs ranged between 192 mg and 227 mg, with an average muscle mass of 208 mg (Figure 5). In contrast, mice from the control group exhibited comparatively atrophied quadriceps with an average mass of 111 mg. Quadriceps harvested from mice that received 250 nM of tegaserod weighed an average of 165 mg and were noticeably less atrophied than control group. Quadriceps muscles from mice receiving 2,500 nM tegaserod were similarly larger than control at 127 mg, but failed to reach statistical significance. Thus, tegaserod at the lower dose resulted in an increased average quadriceps muscle mass presumably due to more successful reinnervation and/or better protection from atrophy in comparison to control.

### EXAMPLE 7: Histology

At the end of the recovery period, femoral nerves were fixed and histology was performed to assess nerve morphology within the regenerated region 1 mm into the 2 mm gap. Animals were perfused with 4% paraformadehyde and explanted nerves were post-fixed in osmium tetroxide and embedded in resin. One and 2 µm thick sections were cut into the center of the regenerated nerve and sections were stained with 1% toluidine blue and 1% borax. Sections were imaged with a Zeiss Axiocam, using 20x, 40x, and 100x objectives and AxioVision imaging software. ImageJ software was used to calculate total nerve area, number of axons, % of myelinated axons, and g-ratio as described Mehanna et al. (2009), see above.

Results: In comparison to unsevered nerves, regenerated nerves following injury showed a lesser-organized nerve structure, with fibrous tissue surrounding the nerve within the conduit, although with the nerve cables appearing relatively contiguous and not heavily infiltrated by fibrous tissue (Figure 6A-D). Quantification of the number of axons within the regenerated nerves showed that the number of axons present within these areas did differ significantly. Unsevered control nerves had an average of 608 axons whereas regenerated nerves from the vehicle control, 250 nM tegaserod, and 2,500 nM tegaserod had averages of 432, 589 and 434 axons, respectively. Thus, at the lower dose of tegaserod the number of axons was increased within the regenerated nerves (Figure 6E).

Assessment of myelination in the regenerated nerves similarly showed improvements in nerves treated with 250 nM tegaserod (Figure 6F). While nearly 100% of axons from the unsevered nerves showed compact myelin around the axons, over 20% of axons from the vehicle control treated group were unmyelinated (78% myelinated axons). Tegaserod treatment of 250 nM correlated with 88% myelinated axons, while axons from the 2,500 nM treatment group were 83% myelinated. When the g-ratios of the myelinated axons were quantified, we found that all conditions promoted overlapping ranges of g-ratios with no observable differences between groups (data not shown).

## Claims

1. Tegaserod or a pharmaceutically acceptable salt or solvate thereof for use in a method of treating a disease or pathological condition which is **characterized by** the loss of functional neurons and/or Schwann cells.

2. Tegaserod or a pharmaceutically acceptable salt or solvate thereof for use in a method according to claim 1, wherein the disease is associated with the loss of neurons and/or Schwann cells in the peripheral nervous system.

3. Tegaserod or a pharmaceutically acceptable salt or solvate thereof for use in a method according to claim 2, wherein the disease or condition is a lesion to the sensory neurons and/or motor neurons.

4. Tegaserod or a pharmaceutically acceptable salt or solvate thereof for use in a method according to claim 1, wherein the disease or condition is associated with the loss of neurons and/or Schwann cells in the central nervous system.

5. Tegaserod or a pharmaceutically acceptable salt or solvate thereof for use in a method according to claim 4, wherein the disease or condition is a lesion to the brain and/or spinal cord.

6. Tegaserod or a pharmaceutically acceptable salt or solvate thereof for use according to any of claims 3 or 5, wherein the lesion results from a surgical procedure or trauma.

7. Tegaserod or a pharmaceutically acceptable salt or solvate thereof for use according to claim 1, wherein the disease is a neurodegenerative disease.

8. Tegaserod or a pharmaceutically acceptable salt or solvate thereof for use according to claim 7, wherein the neurodegenerative disease is selected from the group consisting of Parkinson disease, Alzheimer's disease, Huntington's disease and Multiple Sclerosis.

9. Tegaserod or a pharmaceutically acceptable salt or solvate thereof for use according to claim 1, wherein the disease is a demyelinated disease.

10. Tegaserod or a pharmaceutically acceptable salt or solvate thereof for use according to claim 9, wherein the disease is selected from the group of Charcot-Marie-Tooth disease (CMT), Guillain-Barré syndrome (GBS), Schwannomatosis and chronic inflammatory demyelinating polyneuropathy (CIDP).

11. Pharmaceutical composition comprising tegaserod or a pharmaceutically acceptable salt or solvate thereof for use in a method according to any of claims 1-10.

12. Pharmaceutical composition according to claim 11, wherein said composition comprises the hydrogen maleate salt of tegaserod.

13. Pharmaceutical composition according to claim 11 or 12, wherein the composition is formulated for administration to the peripheral or central nervous system by oral administration, injection, or infusion.

14. Pharmaceutical composition according to any of claims 11-12, wherein the composition is formulated for local administration to a site of nerve injury.

15. Pharmaceutical composition according to claim 14, wherein said carrier is a hydrogel.
